# FASCICULE DE BREVET EUROPEEN

(11) **EP 0 805 652 B1**
(45) Date de publication et mention de la délivrance du brevet: **16.10.2002**
(21) Numéro de dépôt: 96939989.8
(22) Date de dépôt: 27.11.1996
(51) Int. Cl.: A61B 10/00

(54) **DISPOSITIF POUR LE PRELEVEMENT DE MUQUEUSES UTERINES**
VORRICHTUNG ZUR ENTNAHME VON MUKOSA AUS DER GEBÄRMUTTER
DEVICE FOR COLLECTING ENDOMETRIAL FRAGMENTS

(30) Priorité: 27.11.1995 FR 9514018; 25.06.1996 FR 9607852
(43) Date de publication de la demande: 12.11.1997
(73) Titulaire: LABORATOIRE C.C.D., 75008 Paris (FR)
(72) Inventeur: CORNIER, Edgard, F-92200 Neuilly (FR)
(74) Mandataire: Wagret, Frédéric
(86) Numéro de dépôt international: FR9601881
(87) Numéro de publication internationale: WO97019642

(56) Documents cités:
- EP-A- 0 283 929
- DE-A- 3 732 582
- FR-A- 2 602 414
- GB-A- 1 573 819
- US-A- 3 088 454
- US-A- 3 554 185
- US-A- 4 777 947

## Description

La présente invention concerne un dispositif apte à permettre de prélever des fragments de la paroi interne de l'utérus, tels que des fragments de muqueuses.

Ce type de dispositif comprend généralement un tube cylindrique, d'un diamètre extérieur d'environ 3 millimètres et de diamètre interne de 1,5 à 2,6 millimètres, pour une longueur d'environ 25 centimètres. Le tube est ouvert à une première extrémité (extrémité proximale) et comporte à son extrémité opposée (extrémité distale) un trou, d'un diamètre d'environ 2 millimètres, dit trou d'aspiration et prévu sur la paroi cylindrique du tube, c'est-à-dire dans un plan parallèle à l'axe longitudinal du tube. A l'intérieur de ce dernier, est apte à coulisser un piston fixé à l'extrémité distale d'une tige, tandis que l'autre extrémité (proximale) de la tige est solidaire d'un organe de préhension.

Ce type de dispositif est plus connu sous la dénomination commerciale PIPELLE DE CORNIER (marque déposées), (voir par exemple le document FR-A-2 602 414).

L'utilisation, en général à usage unique, de ce dispositif connu est la suivante : celui-ci est introduit à travers le col de la patiente, jusqu'au fond utérin. Des graduations prévues sur le tube permettent de localiser l'arrivée de l'extrémité distale du tube (pourvu du trou), au début de la cavité utérine. L'opérateur, tout en maintenant le tube en tirant sur la tige, par l'organe de préhension, dans le sens de l'éloignement par rapport à la patiente, réalise une dépression à l'intérieur du tube, et donc un phénomène d'aspiration au niveau du trou disposé à l'extrémité distale du tube. Le prélèvement de fragments de la paroi utérine et de la muqueuse utérine est réalisé en déplaçant le tube, de préférence par mouvement de va-et-vient longitudinal, et de rotation autour de l'axe longitudinal, tout en maintenant l'extrémité distale du tube contre la paroi. Des fragments de muqueuses se trouvent donc arrachés de la paroi et sont aspirés dans le tube au travers du trou d'aspiration. Ce dernier, en vue de côté, dans un plan transversal à l'axe du trou, présente une concavité tournée vers l'extérieur du tube. En d'autres termes, toujours en vue de côté, les bords du trou forment une cuvette dont la concavité est tournée vers l'extérieur du tube.

Une fois l'opération de prélèvement effectuée, l'opérateur retire le dispositif et ensuite déverse le contenu du tube résultant des prélèvements, dans un récipient contenant un liquide de fixation histologique.

On comprend que ce type de dispositif doit permettre de prélever des fragments de parois (muqueuses) utérines, de manière fiable, et bien entendu sans douleur. Également, le prélèvement doit être représentatif et donc régulier, en terme de profondeur, dans un plan transversal à la paroi. Le prélèvement doit être également aisé et rapide pour raccourcir au maximum l'opération de prélèvement, compte tenu des désagréments qu'elle comporte pour la patiente.

Les dispositifs connus et mentionnés ci-dessus donnent relativement satisfaction.

Cependant, ils sont susceptibles d'amélioration, et c'est un des objets de la présente invention de proposer un dispositif de prélèvement perfectionné présentant des conditions d'utilisation améliorées, notamment en termes de fiabilité et de conditions de prélèvement optimales, tout en respectant les conditions requises et rappelées ci-dessus.

A cet effet, selon l'invention, le dispositif de prélèvement de fragments de parois d'organes internes, tels que la paroi utérine, du type comportant :
◇ un tube dont l'extrémité proximale (la plus éloignée de la patiente) est ouverte et dont l'extrémité opposée (distale) est obturée, à l'exception d'un trou dit d'aspiration ;
◇ un piston étanche apte à se déplacer dans ledit tube et relié à l'extrémité distale d'une tige dont l'extrémité proximale (opposée à la patiente) est pourvue d'un organe de préhension ;
est caractérisé en ce que la surface de la paroi extérieure du tube, à proximité dudit trou, sur au moins une partie de la périphérie de ladite paroi, présente une rugosité supérieure au reste du tube.

Avantageusement, l'extrémité du tube présente une surface rugueuse sur la périphérie dudit tube et de préférence sur une longueur, dans le sens longitudinal du tube, comprise entre 2 et 50 millimètres, et de préférence entre 5 et 20 millimètres.

Selon une première forme de réalisation, la surface rugueuse de l'extrémité du tube est traitée par frottement avec un matériau abrasif.

Selon une autre forme de mise en oeuvre, une partie au moins de la surface rugueuse de l'extrémité du tube est soumise à une attaque chimique, de manière à créer des micro-porosités en surface, ou équivalent, pour atteindre le degré de rugosité voulu.

De manière avantageuse, la surface traitée en vue d'augmenter la rugosité de la surface extérieure du tube, est disposée en deçà du trou d'aspiration, par rapport à l'extrémité distale dudit tube.

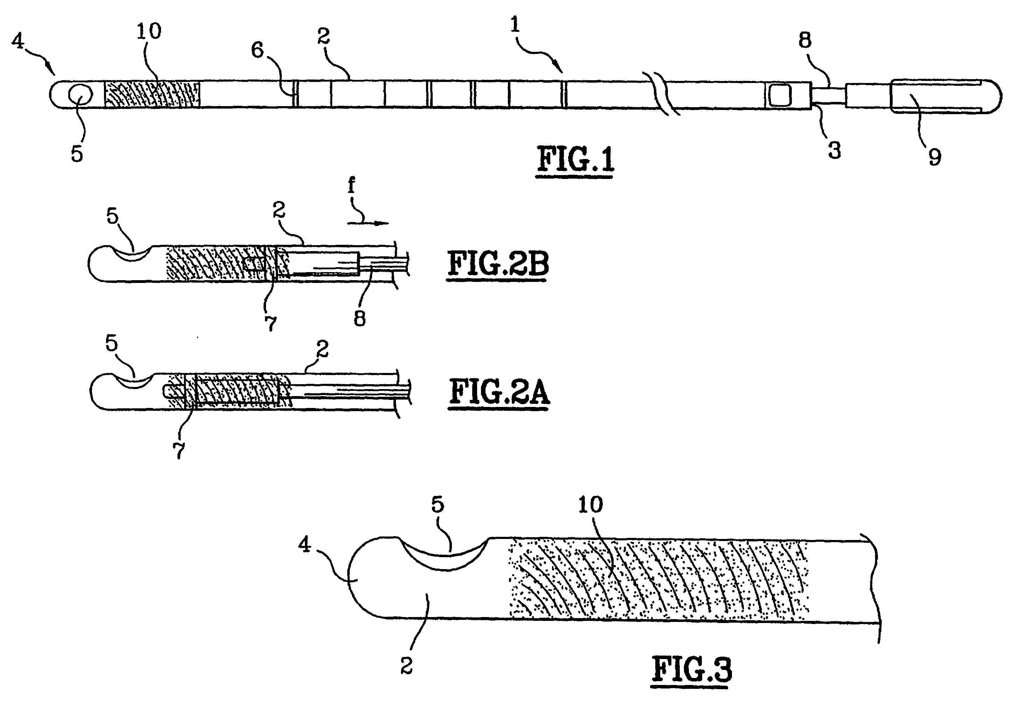

L'invention sera bien comprise à la lumière de la description qui suit se rapportant à un exemple illustratif et non limitatif, en référence au dessin annexé dans lequel :
◇ la figure 1 est une représentation en plan du dispositif de prélèvement ;
   Les figures 2A et 2B montrent l'extrémité distale du tube pour deux positions différentes du piston ; et
◇ la figure 3 est une représentation schématique, à échelle agrandie, de l'extrémité distale du tube.

Comme montré sur la figure 1, le dispositif de prélèvement portant la référence générale 1, comprend un tube cylindrique 2, à base circulaire, et comportant une ouverture 3 à l'une de ses extrémités, à savoir l'extrémité proximale, en d'autres termes celle la plus éloignée de la patiente. L'extrémité opposée, ou extrémité distale, et référencée 4, est obturée, à l'exception d'un trou d'aspiration 5, de section de préférence circulaire.

Le diamètre extérieur du tube 2 est compris entre 2 et 4 millimètres et, selon un exemple particulier, égal à 3,14 millimètres, et le diamètre intérieur du tube est de 2,6 millimètres par exemple. Toujours à titre illustratif, le tube présente une longueur de 20 à 30 centimètres, et par exemple de 23,5 centimètres. Le diamètre du trou est d'environ 2 millimètres, et de préférence de 2,1 mm.

Le tube 2 comprend des marques ou repères, dont l'un d'entre eux porte la référence 6, disposés dans des plans transversaux à l'axe longitudinal du tube.

En référence aux figures 2A et 2B, à l'intérieur du tube 2, est susceptible de coulisser un piston 7, sous la forme d'une bague ou rondelle, de diamètre extérieur sensiblement équivalent au diamètre intérieur du tube. Le piston 7 est solidaire de l'extrémité distale d'une tige 8 dont l'extrémité proximale (du côté de la patiente) est solidaire d'une poignée 9 (figure 1).

En partant de la position de départ représentée sur la figure 2A, l'opérateur, tout en maintenant le tube 2, tire sur la poignée 9, dans le sens de la flèche f (figure 2B); la tige 8 et le piston 7 se trouvent entraînés dans la même direction (vers la patiente). Ce faisant, il se crée une dépression au niveau du trou d'aspiration 5.

L'aspiration ainsi créée au niveau du trou 5, entraîne dans le tube 2 les muqueuses détachées de la paroi utérine où le dispositif est introduit. Les repères 6 permettent, de manière connue, de s'assurer de l'arrivée de l'extrémité distale 4, au début de l'utérus. De manière traditionnelle, le dispositif est déplacé, par mouvement de va-et-vient de direction longitudinale, combiné avec un mouvement de rotation, dans la cavité utérine, afin de permettre le déplacement du trou d'aspiration 5 par rapport à la paroi utérine d'où sont prélevées les muqueuses.

La figure 3 montre une forme de réalisation de l'invention, à plus grande échelle, et plus précisément la partie d'extrémité distale 4 du tube 2, où est disposé le trou d'aspiration 5.

Une zone 10, représentée en grisé/hachuré sur le tube 2, correspond à un état de surface particulier du tube 2, sur sa paroi extérieure. L'état de surface de cette zone est tel qu'il présente des propriétés de rugosité supérieures au reste de la surface du tube 2.

Le tube 2 est, de préférence, dans la zone 10, traité en surface sur une longueur d'environ 10 millimètres et, de préférence encore, sur la totalité du pourtour du tube.

Le traitement de surface peut être effectué par grattage, polissage, ou tout autre opération similaire, à l'aide d'un matériau abrasif, et apte à créer des micro-rugosités ou micro-pores. La zone rugueuse 10 est de préférence disposée à proximité du trou d'aspiration 5.

L'état de surface rugueux de la zone 10 peut être également effectué par attaque chimique du matériau constituant le tube 2.

Le tube 2 est avantageusement réalisé en matière plastique transparente, telle que du polypropylène. Le piston est en EVA, tandis que la tige 8 est par exemple en résine d'acétal souple.

Le dispositif de l'invention est, de préférence, à usage unique et peut présenter toute variation en terme de forme et de dimension, sans sortir du cadre de l'invention.

La présente invention a pour objet un dispositif de prélèvement endo-utérin à usage unique.

Le dépistage systématique, par frottis, du cancer du col de l'utérus a permis de réduire, dans une large mesure, le taux de mortalité qui lui était imputable en favorisant son diagnostic précoce.

Dans ce but, les gynécologues ont depuis déjà de nombreuses années pris l'habitude de surveiller régulièrement leurs patientes en prélevant, par raclage, des cellules au niveau de leur col utérin au moyen de spatules, bâtonnets, coton tiges, ... avant de les étaler sur des lames de façon à permettre leur analyse.

De telles analyses ne s'avèrent cependant pas toujours suffisantes, et ne permettent, en particulier pas de détecter ou de confirmer un diagnostic de cancer de l'endomètre. Il est en effet indispensable dans ce but d'effectuer des prélèvements au niveau de la muqueuse épithéliale, donc à la partie interne de la cavité utérine, notamment pour permettre une étude à visée histologique.

Pendant longtemps, de tels prélèvements ne pouvaient être effectués que par curetage, sous anesthésie générale.

Depuis quelques années, on a créé un dispositif à usage unique spécialement adapté à de tels prélèvements qui a immédiatement rencontré un grand succès auprès des praticiens.

Ce dispositif, connu sous le nom de «Pipelle de Cornier», est schématiquement constitué par un tube de prélèvement souple et lisse destiné à être introduit dans la cavité utérine et par un piston mobile à l'intérieur de ce tube et relié par une tige à bouton d'actionnement faisant saillie par l'extrémité proximale ouverte du tube. L'extrémité distale fermée et arrondie de ce tube, dont le diamètre est de l'ordre de 3mm, est percée d'un orifice, et en règle générale d'un orifice latéral d'environ 2mm de diamètre; cet orifice permet l'aspiration, à la partie interne du tube, de la muqueuse utérine sous l'action de la dépression créée par un déplacement du piston en direction de l'extrémité proximale, suite au tirage du bouton d'actionnement effectué par le praticien.

La muqueuse ainsi arrachée est introduite à la partie interne du tube de prélèvement, puis transférée après extraction du dispositif, dans un flacon contenant un liquide de conservation, à l'aide du piston après sectionnement du tube au niveau de son extrémité distale, ce en vue de son étude histologique.

Malgré ses avantages certains, ce dispositif présente l'inconvénient de ne pas permettre de gratter le revêtement endo-utérin lorsque la muqueuse utérine ne se détache pas, comme c'est le cas lorsqu'une atrophie existe ou chez les patientes ménopausées.

Pour remédier à cet inconvénient, on a déjà proposé d'adjoindre à l'extrémité du dispositif de prélèvement endo-utérin divers types de brosses (voir par exemple les documents US-A-3 088 454 et GB-A-1 573 819) permettant le grattage et le détachement des cellules épithéliales pour permettre leur étude cytologique; l'adjonction de telles brosses présente toutefois, l'inconvénient de fragiliser le dispositif, et en outre, d'augmenter notablement le diamètre du tube de prélèvement, et par voie de conséquence de nuire au confort de la patiente.

La présente invention a pour objet de proposer un dispositif de prélèvement endo-utérin à usage unique du type susmentionné qui soit susceptible de remédier à ces inconvénients tout en permettant d'effectuer, parallèlement au prélèvement histologique habituel, un prélèvement à visée cytologique de nature à venir compléter celui-ci lorsqu'il n'est pas possible de détacher la muqueuse utérine.

A cet effet, l'invention concerne un dispositif de prélèvement endo-utérin à usage unique caractérisé en ce que la surface externe du tube de prélèvement est rugueuse au niveau de son extrémité distale de façon à permettre un double prélèvement par raclage des cellules épithéliale à ce niveau en plus du prélèvement histologique.

Selon l'invention, la rugosité s'étend de préférence sur la totalité de la périphérie de l'extrémité distale du tube de prélèvement, ce sur une longueur d'environ 1 à 1,5 cm. Celle-ci peut avantageusement être obtenue par une opération de rodage à ce niveau du matériau (en règle générale de la matière plastique) constitutif du tube de prélèvement.

Le fait de rendre rugueuse, en particulier de roder, l'extrémité distale du type de prélèvement permet de créer un frottement susceptible de détacher les cellules épithéliales; les cellules ainsi détachées peuvent ensuite être aspirées sous l'effet d'une dépression induite par le piston puis étalées sur une lame de façon à obtenir un prélèvement endo-utérin cytologique comparable au frottis du col de l'utérus; on peut également analyser ainsi les cellules restées attachées à la zone rugueuse.

Compte tenu de ce qui précède, l'avantage essentiel du dispositif de prélèvement endo-utérin conforme à l'invention, dont la mise en oeuvre est totalement indolore pour la patiente, est donc de permettre d'effectuer aussi bien un prélèvement histologique qu'un prélèvement cytologique; en effet, après sectionnement du tube de prélèvement au niveau de son extrémité distale à l'aide de ciseaux, le contenu de celui-ci peut soit être étalé sur une lame, si le prélèvement est à but cytologique, soit être placé dans un liquide de conservation, si le prélèvement est à but histologique, soit les deux en étalant la partie rodée sur des lames à visée cytologique après avoir repoussé à l'aide du piston le contenu du tube pour étude histologique.

Les caractéristiques du dispositif de prélèvement endo-utérin qui fait l'objet de l'invention seront décrite plus en détail en se référant aux dessins annexés dans lesquels :
- la figure 4 représente le dispositif,
- les figures 5 et 6 sont des schémas montrant comment divers types d'analyse peuvent être effectués à partir d'un tel dispositif.

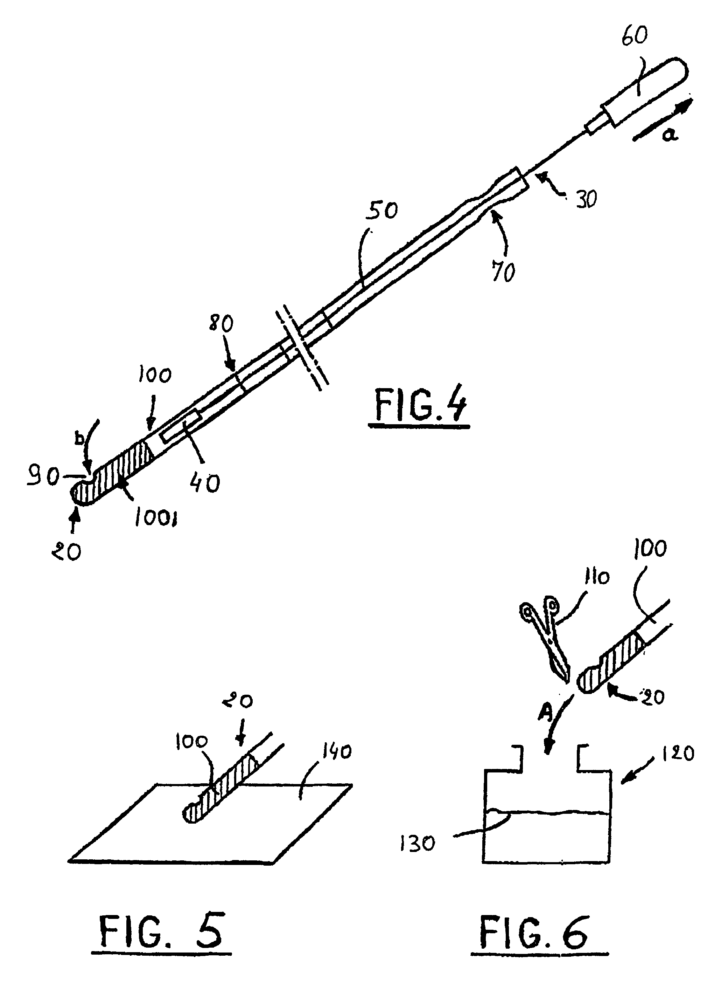

Selon la figure 4, le dispositif est essentiellement constitué par un tube de prélèvement 100 réalisé en une matière transparente souple et lisse et ayant une longueur de l'ordre de 23 cm et un diamètre de l'ordre de 3 mm.

Ce tube 100 présente une extrémité distale arrondie et fermée 20 par laquelle il est destiné à être introduit dans la cavité utérine et une extrémité proximale ouverte 30.

Un piston 40, mesurant environ 1 cm de longueur peut coulisser à la partie interne du tube de prélèvement 100. Ce piston 40 est relié par une tige 50 à un bouton d'actionnement 60 faisant saillie par l'extrémité proximale ouvertue 3 du tube de façon à permettre au praticien de déplacer le piston 40.

Une partie aplatie 70 prévue au niveau de l'extrémité proximale 30 du tube 100 permet d'empêcher toute sortie intempestive du piston 40.

Des graduations de repérage 80 situées sur la périphérie du tube de prélèvement 100 notamment à 4,5,6,7,8,9 et 10 cm de son extrémité distale 2, permettent de mesurer la profondeur de l'utérus.

L'extrémité distale 20 du tube 100 est, par ailleurs, munie d'un orifice d'aspiration latéral 90 mesurant environ 2 mm de diamètre ainsi que d'une partie rodée 1001 s'étendant sur lune longueur de 1 à 1,5 cm sur toute la périphérie du tube de prélèvement 100. Cette partie rodée 1001 est représentée en hachuré sur les figures.

### Le mode d'utilisation de ce dispositif est le suivant :

Après désinfection du col de l'utérus, et en-dehors des contre-indications habituelles (grossesse, infection), le tube de prélèvement 100 est poussé doucement jusqu'au fond de l'utérus. Il est à noter que le passage du col est habituellement indolore pour la patiente. Préalablement à cette introduction, le praticien peut, le cas échéant, plier le tube afin de le précourber.

Lors de l'introduction, le piston 40 doit être situé au niveau de l'extrémité distale 20 du tube 100.

Lorsque celui-ci a été placé au fond de la cavité utérine, le praticien crée une dépression en tirant, selon la flèche a, sur le bouton d'actionnement 6, et par suite, en déplaçant le piston 40 vers l'extrémité 30 du tube 100.

Des fragments tissulaires détachés de l'endomètre peuvent ainsi être aspirés selon la flèche b à la partie interne du tube 100.

Lorsque cette première phase a été effectuée, le praticien déplace le tube 100 par des mouvements circulaires hélicoïdaux depuis le fond jusqu'au bord de l'utérus de sorte que la partie rodée 1001 vienne exercer une action de frottement et de grattage circulaire susceptible de détacher des cellules épithéliales; celles-ci peuvent ensuite être aspirées à la partie interne du tube 1 sous l'action de la dépression induite par le tube 40; il s'ajoute donc au prélèvement histologique par aspiration et détachement de la muqueuse utérine, une fonction de grattage de l'épithélium.

Le praticien retire ensuite le tube 100 de l'utérus et le sectionne au niveau de l'extrémité distale 20 à l'aide de ciseaux 110 comme représenté schématiquement sur la figure 5. Le contenu du tube est alors repoussé à l'aide du piston 40 à partir du bouton d'actionnement 60, et est soit, comme représenté sur la figure 6, introduit selon la flèche A dans le flacon 120 contenant un liquide de conservation 130 lorsque le prélèvement est à but histologique, soit étalé sur une lame si le prélèvement est à but cytologique.

L'invention permet également de réaliser à la fois ces deux types d'analyse en repoussant la partie interne du tube 100 dans un flacon 120, comme représenté sur la figure 5, et en étalant la partie rodée externe 1001 sur une lame 140 comme représenté sur la figure 5, d'une manière largement comparable à celle permettant de réaliser des frottis du col de l'utérus.

## Revendications

1. Dispositif de prélèvement de fragments de parois d'organes internes, tels que la paroi utérine, du type comportant:
un tube (2) dont l'extrémité proximale c'est-à-dire la plus éloignée du patient est
ouverte et dont l'extrémité (4) opposée est obturée à l'exception d'un trou (5) dit d'aspiration;
un piston (7) étanche apte à se déplacer dans ledit tube (2) et relié à l'extrémité distale d'une tige (8) dont l'extrémité proximale c'est-à-dire opposée au patient est pourvue d'un organe (9) de préhension;
des moyens (10) prévus du côté de l'extrémité (4) distale du tube (2), et à proximité du trou (5) d'aspiration,
**caractérisé en ce que** la surface (10) de la paroi extérieure du tube, à proximité dudit trou (5), sur au moins une partie de la périphérie de ladite paroi, présente une rugosité supérieure au reste du tube.

2. Dispositif selon la revendication 1, **caractérisé en ce que** l'extrémité du tube présente ladite surface (10) rugueuse sur la périphérie dudit tube (2) et de préférence sur une longueur, dans le sens longitudinal du tube, comprise entre 2 et 50 millimètres, et de préférence entre 5 et 20 millimètres.

3. Dispositif selon l'une des revendications 1 ou 2, **caractérisé en ce que** ladite surface rugueuse (10) est réalisée par frottement du tube (2) avec un matériau abrasif.

4. Dispositif selon l'une des revendications 1 ou 2, **caractérisé en ce que** ladite surface (10) rugueuse est réalisée par attaque chimique, de manière à créer une micro-porosité en surface, ou équivalent, pour atteindre le degré du rugosité voulu.

5. Dispositif selon l'une des revendications précédentes, **caractérisé en ce que** ladite surface (10) rugueuse est disposée en deçà du trou (5) d'aspiration, par rapport à l'extrémité (4) distale dudit tube.

## Patentansprüche

1. Vorrichtung zur Entnahme von Wand-Fragmenten von inneren Organen, wie von der Uterus-Wand, des Typs, der folgendes aufweist:
ein Rohr (2), dessen proximales Ende (1), d.h. das am weitesten vom Patienten entfernt ist, offen ist, und dessen gegenüberliegendes Ende (4), mit Ausnahme eine Lochs (5), das Ansaugloch genannt wird, verschlossen ist;
einen dichten Kolben (7), der zur Verschiebung in Rohr (2) ausgelegt ist und der am distalen Ende mit einer Stange (8) verbunden ist, deren proximales Ende (1), d.h. gegenüber dem Patienten, zur Organentnahme vorgesehen ist;
Mittel (10), die auf der Seite des distalen Endes (4) des Rohrs (2) und in der Nähe des Ansauglochs (5) vorgesehen sind,
**dadurch gekennzeichnet, dass** die Oberfläche (10) der Außenwand des Rohrs in der Nähe des Lochs (5) auf mindestens einem Teil der Peripherie der Wand eine Rauheit aufweist, die größer ist als auf dem restlichen Rohr.

2. Vorrichtung nach Anspruch 1, **dadurch gekennzeichnet, dass** das Ende des Rohrs die raue Oberfläche (10) auf der Peripherie des Rohrs (2) und vorzugsweise auf einer Länge in Längsrichtung des Rohrs zwischen 5 und 20 mm, vorzugsweise zwischen 5 und 20 mm, aufweist.

3. Vorrichtung nach Anspruch 1, **dadurch gekennzeichnet, dass** die raue Oberfläche (10) durch Reiben des Rohrs (2) mit einem abrasiven Material hergestellt ist.

4. Vorrichtung nach einem der Ansprüche 1 oder 2, **dadurch gekennzeichnet, dass** die raue Oberfläche (10) durch einen chemischen Angriff hergestellt ist, derart, dass eine Oberflächen-Mikroporosität oder Ähnliches erzeugt wird, um den gewünschten Rauheitsgrad zu erzielen.

5. Vorrichtung nach einem der vorhergehenden Ansprüche, **dadurch gekennzeichnet, dass** die raue Oberfläche (10) bezüglich des distalen Endes (4) des Rohrs oberhalb des Ansauglochs (5) angeordnet ist.

## Claims

1. Device for collecting fragments of walls of internal organs, such as the endometrium, of the type comprising:
a tube (2) of which the proximal end, i.e. the one furthest away from the patient, is open and of which the opposite end (4) is obturated with the exception of a so-called suction hole (5);
a tight piston (7) adapted to move in said tube (2) and connected to the distal end of a rod (8) of which the proximal end, i.e. opposite the patient, is provided with a member (9) for gripping;
means (10) provided towards the distal end (4) of the tube (2), and in the vicinity of the suction hole (5),
**characterized in that** the surface (10) of the outer wall of the tube, in the vicinity of said hole (5), over at least a part of the periphery of said wall, presents a roughness greater than the rest of the tube.

2. Device according to Claim 1, **characterized in that** the end of the tube presents said rough surface (10) over the periphery of said tube (2) and preferably over a length, in the longitudinal direction of the tube, included between 2 and 50 millimeters, and preferably between 5 and 20 millimeters.

3. Device according to one of Claims 1 or 2, **characterized in that** said rough surface (10) is made by rubbing the tube (2) with an abrasive material.

4. Device according to one of Claims 1 or 2, **characterized in that** said rough surface (10) is made by chemical attack, so as to create a micro-porosity on the surface, or equivalent, in order to attain the desired degree of roughness.

5. Device according to one of the preceding Claims, **characterized in that** said rough surface (10) is formed this side of the suction hole (5) with respect to the distal end (4) of said tube.
